Europäisches Patentamt

European Patent Office

Office européen des brevets

(19)

(11) EP 0 804 736 B1

(12) FASCICULE DE BREVET EUROPEEN

(45) Date de publication et mention
de la délivrance du brevet:
**31.03.1999 Bulletin 1999/13**

(51) Int Cl.$^6$: **G01N 33/68**, G01N 33/577,
G01N 33/58

(21) Numéro de dépôt: **96901825.8**

(22) Date de dépôt: **19.01.1996**

(86) Numéro de dépôt international:
**PCT/FR96/00095**

(87) Numéro de publication internationale:
**WO 96/22535 (25.07.1996 Gazette 1996/34)**

(54) **PROCEDE DE DOSAGE ULTRASENSIBLE DE LA TROPONINE I CARDIAQUE**

VERFAHREN ZUR HOCHEMPFINDLICHEN DOSIERUNG VON HERZTROPONIN - I

ULTRASENSITIVE PROCESS FOR ASSAYING CARDIAC TROPONINE I

(84) Etats contractants désignés:
**AT BE CH DE DK ES FR GB GR IE IT LI LU MC NL
PT SE**

(30) Priorité: **19.01.1995 FR 9500597**

(43) Date de publication de la demande:
**05.11.1997 Bulletin 1997/45**

(73) Titulaire: **PASTEUR SANOFI DIAGNOSTICS
92430 Marnes La Coquette (FR)**

(72) Inventeurs:
• **CALZOLARI, Charles
F-30220 Aigues-Mortes (FR)**
• **FLECHEUX, Odile
F-78220 Viroflay (FR)**
• **PAU, Bernard
F-34080 Montpellier (FR)**

(74) Mandataire: **Le Guen, Gérard et al
CABINET LAVOIX
2, place d'Estienne d'Orves
75441 Paris Cédex 09 (FR)**

(56) Documents cités:
**EP-A- 0 116 454          WO-A-90/00168
WO-A-93/13405          WO-A-94/27156**

• **CLINICAL CHEMISTRY, vol. 39, no. 6, Juin 1993,
WINSTON US, pages 972-979, XP002003381 C.
LARUE ET AL.: "Cardiac-specific
immunoenzymometric assay of Troponin I in the
early phase of acute myocardial infarction." cité
dans la demande**
• **BIOCHEMICAL SOCIETY TRANSACTIONS, vol.
15, no. 6, Décembre 1987, LONDON, GB., pages
1060-1061, XP002003382 B. CUMMINS ET AL.:
"Immunoassay of the cardiac-specific isoform of
troponin-I in the diagnosis of heart muscle
damage. "**
• **CLINICAL CHEMISTRY, vol. 38, no. 11,
Novembre 1992, WINSTON US, pages 2203-2214,
XP002003383 G. S. BODOR ET AL.:
"Development of monoclonal antibodies for an
assay of cardiac troponin-I and preliminary
results in suspected cases of myocardial
infarction. " cité dans la demande**

EP 0 804 736 B1

**Description**

La présente invention concerne un procédé de dosage ultrasensible de la troponine I cardiaque, effectué par chimiluminescence.

L'infarctus du myocarde (IDM) représente toujours une urgence médicale et demeure la principale cause de mortalité et de morbidité cardio-vasculaire dans le monde.

Dans tous les cas, le traitement instauré a pour but de protéger le tissu myocardique de la nécrose irréversible. De nouveaux moyens thérapeutiques, comme les thrombolytiques ou l'angioplastie de première intention, permettent aujourd'hui de restaurer rapidement la circulation coronarienne, de façon à limiter la taille de la zone infarcie et de réduire l'incidence de la mortalité et de la morbidité.

Ces outils thérapeutiques sont d'autant plus efficaces qu'ils sont mis en oeuvre au plus tôt après le début des manifestations cliniques. Les cliniciens confrontés à cette pathologie doivent pouvoir disposer d'outils diagnostiques de plus en plus performants.

Le dosage des protéines sériques et en particulier des enzymes est devenu un élément déterminant pour le diagnostic de l'infarctus du myocarde et pour l'appréciation du succès ou de l'échec d'une reperfusion.

On sait que la troponine est un complexe protéique myofibrillaire, constitué de 3 protéines, les troponines C, I et T.

La troponine I cardiaque est une protéine contractile exclusivement présente dans le muscle cardiaque [Wilkinson J.M. et al, Nature (1978), 271, p. 31-35 ; Wade R. et al, Genomics (1990), 7, p. 346-357].

Son rôle physiologique est d'inhiber, en l'absence de calcium, l'activité ATPasique du complexe actine-myosine et donc d'empêcher la contraction musculaire [Perry S.V., Biochem. Soc. Trans (1979), 7, p. 593-617].

Lors de l'infarctus dû à l'obstruction de l'une des artères nourricières du coeur (artère coronaire), il apparaît une ischémie puis une nécrose myocardique qui se traduit par une destruction des cellules cardiaques et la libération de la troponine I cardiaque dans le sang. La troponine I représente donc un marqueur très spécifique de l'infarctus du myocarde.

Ainsi on a récemment préconisé le dosage de la troponine I pour le diagnostic précoce de l'infarctus du myocarde [Am. Heart J. (1981), 110, p. 1334-1344; Molecular Immunology (1992), 29 (2), p. 271-278].

De plus, il est désormais acquis que chez certains patients l'angor instable - qui représente une étape critique de l'ischémie myocardique -, peut être associé à un haut risque de survenue d'infarctus du myocarde ou de mort subite. Les études *post-mortem* sur cette catégorie de patients ont montré que l'apparition de ces complications était très souvent précédée de micro-infarctus cardiaques [Davies M.J. et al, Circulation (1985), 71, p. 699-708].

Le dosage de la troponine I cardiaque semble donc être particulièrement intéressant dans la détection des sujets à risque, chez les patients atteints d'angor instable, plus spécialement pour le diagnostic des micro-infarctus.

Par ailleurs, le dosage de la troponine I peut être envisagé lors de la surveillance d'un traitement thrombolytique pour confirmer le succès ou l'échec de la reperfusion, comme aussi lors du suivi postopératoire des interventions en chirurgie cardiaque (pontages coronariens, angioplasties etc.). Le dosage de la troponine I trouve aussi son application dans le diagnostic des rejets de greffes cardiaques après transplantation et le suivi des thérapeutiques cardiotoxiques utilisant par exemple des anthracyclines.

On connaît déjà des méthodes immunoenzymatiques permettant la détermination de la troponine I. Larue C. et al [Mol. Immunol. (1992), 29(2), p. 271-278 ; Clin. Chem. (1993), 39/6, p. 972-979] décrivent un procédé de dosage immunoenzymatique de type sandwich qui utilise pour la révélation enzymatique le substrat chromogène tetraméthylbenzydine (TMB)- $H_2O_2$. Après la révélation, la lecture de l'absorbance est faite à 450 nm et la limite de détection, selon les auteurs, est de 0,2 µg/l.

Un autre procédé immunoenzymométrique de la troponine I décrit par Bodor et al permet de détecter cette dernière à des concentrations de l'ordre de 1,5 à 3,1 µg/l [Bodor et al, Clin. Chem. (1992), 38/11, p. 2203-2214, Adams J. et al, Circulation (1993), 88(1), p. 101-106]. Ce dosage utilise pour la révélation enzymatique un substrat chromogène de la phosphatase alcaline, le p-nitrophénylphosphate, et la mesure est effectuée à 405 nm.

Grâce à ces dosages, il a été possible de mettre en évidence la présence de la troponine I dans le plasma des patients, environ 4 heures après le début des manifestations cliniques de l'infarctus du myocarde [Adams J. et al, Circulation (1993), 88(1), p. 101-106].

Toutefois, la sensibilité de ces dosages immunoenzymométriques est largement inférieure à celle obtenue par des dosages basés sur des méthodes très sophistiquées et difficiles à mettre en oeuvre, par exemple des dosages basés sur la mesure de la radioactivité [Am. Heart Journal, (June 1987), 113(6), p, 1333-1334].

On sait aussi que des dérivés cycliques d'hydrazines, notamment les diacylhydrazines, peuvent être utilisés comme réactifs de chimiluminescence [Roswell et al, Methods Enzymol. (1978), 57, p. 409-423]. L'utilisation de ces réactifs a été envisagée ou effectuée lors de certains dosages immunoenzymatiques [Thrope G.H.G. et al, Clin. Chem. (1985), 31, p. 1335-1341; Thrope G.H.G. et al, Medors. Enzymol. (1988), 133, p. 331-353] (Demande WO 88/00695). Par ailleurs, on connaît des méthodes de détection par chimiluminescence mettant en oeuvre la décomposition enzymatique de dérivés du 1,2-dioxetane.

Toutefois, il est connu que la mise en oeuvre de ces réactifs nécessite des recherches intensives puisque l'homme de l'art est confronté à de multiples facteurs limitant pour la mise au point des dosages sensibles (affinité des différents composants de dosage et notamment du système antigène/anticorps, marqueur enzymatique, substrat, principe de détection etc.). En effet, un choix adéquat des différents réactifs et conditions de dosage s'impose. Il est évident que l'on ne peut pas imaginer un dosage sensible et spécifique lorsque le bruit de la mesure (bruit de fond ou bruit de blanc) est important ou lorsque le rapport signal/bruit de la mesure est trop petit. Or justement ce bruit de la mesure dépend des différents réactifs et conditions de dosage choisis.

Il a été maintenant trouvé qu'il était possible, en utilisant un certain type d'anticorps monoclonaux anti-troponine I cardiaque et de substrats adéquats pour la révélation enzymatique, de procéder au dosage par chimiluminescence de la troponine I avec une très grande sensibilité. En effet, en appliquant le procédé de l'invention il est possible d'obtenir une sensibilité de l'ordre de 2 à 5 ng/l, ce qui représente une sensibilité 40 à environ 1500 fois supérieure à celles des procédés immunoenzymométriques de la troponine I les plus sensibles connus jusqu'à présent.

La présente invention concerne un procédé de dosage immunoenzymatique de type sandwich permettant le dosage quantitatif de la troponine I cardiaque caractérisé en ce que les substrats utilisés pour la révélation enzymatique sont des substrats chimiluminescents, choisis parmi un grand nombre de substrats chimiluminescents, en particulier des dérivés d'une diacylhydrazine ou du 1,2-dioxetane.

La présente invention a plus précisément comme objet, un procédé de dosage immunoenzymatique de type sandwich, de la troponine I cardiaque :

- qui met en oeuvre deux anticorps monoclonaux anti-troponine cardiaque dont un est couplé avec un marqueur enzymatique, lesquels anticorps ont, soit naturellement, soit par coopérativité une grande affinité pour la troponine I cardiaque, donc une constante de dissociation à l'équilibre égale ou inférieure à $10^{-8}$ M, de préférence égale ou inférieure à $10^{-9}$ M.

   et

- qui utilise pour la révélation enzymatique un substrat chimiluminescent qui est soit un dérivé d'une diacylhydrazine, soit un dérivé de 1,2-dioxetane.

En effet, la présente invention concerne un procédé de dosage immunoenzymatique quantitatif de type sandwich de la troponine I cardiaque, caractérisé en ce que l'on met en contact les échantillons à doser ou les standards avec un anticorps monoclonal anti-troponine I cardiaque couplé à un marqueur enzymatique et avec un autre anticorps monoclonal anti-troponine I cardiaque, ces anticorps monoclonaux, soit naturellement, soit par effet de coopérativité, ayant pour la troponine I cardiaque une constante de dissociation à l'équilibre égale ou inférieure à $10^{-8}$ M, de préférence égale ou inférieure à $10^{-9}$ M, puis que l'on effectue la révélation enzymatique par addition d'un substrat chimiluminescent choisi et ensuite que l'on procède à la lecture directe du signal lumineux obtenu.

Le procédé de l'invention peut être réalisé soit par un système manuel (kit de dosage de diagnostic) soit par un système automatisé. Selon le mode de réalisation, il est possible d'effectuer soit un dosage en un temps, soit un dosage en deux temps.

Pour le dosage en un temps, les échantillons à doser ou les standards sont mis en contact simultanément avec un anticorps monoclonal anti-troponine I cardiaque couplé à un marqueur enzymatique et avec un deuxième anticorps monoclonal anti-troponine I cardiaque éventuellement fixé sur un support solide. Après incubation et lavage, on procède à la révélation enzymatique. Cette dernière est effectuée selon l'invention par addition d'un substrat chimiluminescent dérivé d'une diacylhydrazine ou du 1,2-dioxetane. La quantité de la troponine I cardiaque présente dans les échantillons à doser ou les standards, est évaluée par la lecture directe du signal lumineux obtenu.

Pour le dosage en deux temps, on met en contact les échantillons à doser ou les standards,

- soit, d'abord avec un anticorps monoclonal anti-troponine I cardiaque couplé à un marqueur enzymatique, puis après incubation, avec un deuxième anticorps monoclonal anti-troponine I cardiaque éventuellement fixé sur un support solide et ensuite on incube de nouveau,
- soit, d'abord avec un anticorps monoclonal anti-troponine I cardiaque éventuellement fixé sur un support solide, puis après incubation et un éventuel lavage, avec un deuxième anticorps monoclonal anti-troponine I cardiaque couplé à un marqueur enzymatique et ensuite on incube à nouveau.

Dans les deux cas, on procède ensuite éventuellement à un lavage, puis à la révélation enzymatique qui est effectuée selon l'invention, par addition d'un substrat chimiluminescent dérivé d'une diacylhydrazine ou du 1,2-dioxetane. La quantité de la troponine I cardiaque présente dans les échantillons à doser ou les standards, est évaluée par la lecture directe du signal lumineux obtenu.

Pour le dosage en deux temps, de manière préférentielle, les échantillons à doser et les standards sont mis en contact d'abord avec un anticorps monoclonal anti-troponine I cardiaque couplé à un marqueur enzymatique puis après incubation, avec un deuxième anticorps monoclonal anti-troponine I cardiaque fixé sur un support solide et ensuite incubés de nouveau.

Comme anticorps monoclonal anti-troponine I cardiaque, on peut utiliser tout anticorps monoclonal ayant une grande affinité pour la troponine I cardiaque et dont la constante de dissociation à l'équilibre est égale ou inférieure à $10^{-8}$ M, de préférence égale ou inférieure à $10^{-9}$ M. On peut aussi utiliser tout couple d'anticorps monoclonaux ayant une liaison coopérative vis à vis de la troponine I cardiaque, cette coopérativité produisant comme résultat une augmentation de l'affinité de l'un des deux anticorps monoclonaux pour la troponine I cardiaque (constante de dissociation à l'équilibre $\leq 10^{-8}$ M) lorsque la troponine I cardiaque est déjà liée avec le deuxième anticorps.

De manière préférentielle, comme anticorps monoclonaux anti-troponine I cardiaque, on peut utiliser les anticorps monoclonaux 11E12 et 8E1 de souris décrits dans Clin. Chem. (1993), 39, p. 972-979. En effet, de manière surprenante il a été constaté que l'anticorps monoclonal 11E12 bloque la libération de la troponine I cardiaque liée à l'anticorps 8E1. Ce phénomène a été mis en évidence en utilisant le système BIACORE™ de PHARMACIA, et en mesurant les constantes cinétiques d'association et de dissociation de la troponine I cardiaque (cTnI) sur l'anticorps monoclonal 8E1, en présence ou en l'absence de l'anticorps 11E12.

Les différents résultats sont donnés dans le Tableau I.

## TABLEAU I

|  | AcM 8E1 : cTnI seule | AcM 8E1 : cTnI+11E12 |
|---|---|---|
| CONSTANTE CINETIQUE DE DISSOCIATION $koff (S^{-1})$ | $3{,}2.10^{-3}$ | $2{,}8.10^{-4}$ |
| CONSTANTE CINETIQUE D'ASSOCIATION $kon (M^{-1} \times S^{-1})$ | $5{,}8.10^{5}$ | $4{,}5.10^{5}$ |
| CONSTANTE DE DISSOCIATION A L'EQUILIBRE $koff / kon (M)$ | $5{,}7.10^{-9}$ | $0{,}6.10^{-9}$ |

Les résultats du Tableau I démontrent que pour l'anticorps 8E1, l'affinité est 10 fois plus élevée si la troponine I cardiaque est en présence de l'anticorps monoclonal 11E12. Plus précisément, la constante de dissociation à l'équilibre est 10 fois plus faible (la constante cinétique d'association ne varie pas). Il existe donc une coopérativité de la liaison des deux anticorps monoclonaux anti-troponine I vis à vis de la troponine I cardiaque. Grâce à cette effet de "blocage" de la dissociation du complexe il est possible de réaliser le procédé de dosage immunoenzymatique, objet de la présente invention, avec ce couple d'anticorps monoclonaux.

Pour la formation du conjugué anticorps monoclonal anti-troponine I cardiaque-marqueur enzymatique on utilise comme marqueur enzymatique soit la phosphatase alcaline soit la peroxydase.

On utilise la phosphatase alcaline lorsque le substrat chimiluminescent est un dérivé de 1,2-dioxetane et la peroxydase lorsque le substrat chimiluminescent est un dérivé d'une diacylhydrazine.

On peut utiliser notamment les anticorps monoclonaux 8E1 ou 11E12 pour préparer les conjugués avec un marqueur enzymatique. De préférence on prépare des conjugués de l'anticorps monoclonal 11E12 avec la peroxydase et des conjugués de l'anticorps monoclonal 8E1 avec la phosphatase alcaline.

Selon le mode de réalisation, on peut envisager comme support solide soit des tubes en polystyrène sur lesquels est fixé l'anticorps anti-troponine I cardiaque (cas du kit de diagnostic), soit des particules magnétiques (notamment ferriques) ou non sur lesquelles est fixé l'anticorps monoclonal anti-troponine I cardiaque (dosage automatisé)

Les conjugués anticorps monoclonal anti-troponine I - marqueur enzymatique sont préparés selon les méthodes connues [J. Histochem Cytochem (1974), 22, p. 1084-1091].

La préparation des anticorps monoclonaux anti-troponine I fixés par exemple par des liaisons covalentes sur une phase solide, est aussi réalisée selon des méthodes décrites dans la littérature [J. Immunological Methods, (1979), 31, p. 231-236].

Lors de la mise en contact des échantillons à doser ou des standards avec un anticorps monoclonal anti-troponine 1 cardiaque et/ou un anticorps monoclonal anti-troponine cardiaque couplé à un marqueur enzymatique, le pH du milieu réactionnel doit être de préférence légèrement acide. Le pH doit notamment être compris entre 5 et 6, plus précisément entre 5,4 et 5,7. Pour ajuster le pH on peut utiliser différents tampons adéquats ou des solutions d'acides organiques faibles, par exemple des solutions ou des tampons d'acide succinique ayant un pH de 5,2 à 5,4.

L'incubation entre le plasma ou le serum et les anticorps anti-troponine I ou les standards, est effectuée entre 20°C et 37°C et le temps d'incubation peut varier entre 5 et 20 minutes.

Après incubation, on procède à un ou plusieurs lavages qui sont effectués à une température de 2° à 37°C, de préférence à une température inférieure à 10°C. Pour les lavages, on utilise de manière préférentielle une solution de tampon phosphate pH 6,8 ou une solution tampon tris pH 8.

Comme il a été indiqué précédemment, comme substrat luminescent on utilise soit un dérivé de diacylhydrazine, plus spécialement le luminol [5-amino-2,3-dihydrophtalazine-1,4-dione], soit un dérivé de 1,2-dioxetane, plus spécialement le lumigen PPD [sel disodique de 4-methoxy 4-(3-phosphatephenyl) spiro (1,2-dioxetane 3-adamantane-2')]. Des substrats luminescents contenant du luminol sont disponibles dans le commerce comme par exemple le luminol ECL-immunoessai signal reagent RPN 190 commercialisé par Amersham ou le luminol BM Chimiluminescence ELISA reagent 1582950 commercialisé par Boehringer Mannheim. On préfère des substrats qui contiennent un mélange de luminol et de 4-iodophénol. Des substrats luminescents contenant du lumigen PPD sont aussi disponibles dans le commerce comme, par exemple, le Lumi-Phos 530 commercialisé par Analytical Luminescence Laboratory. Ce réactif contient du lumigen PPD et un promoteur de la chimiluminescence qui est un tensioactif dérivé de la fluorescéine.

La révélation enzymatique est aussi effectuée à une température comprise entre 20°C et 37°C. La lecture de la chimiluminescence est effectuée de préférence après environ 2-10 minutes.

Pour réaliser la gamme d'étalonnage d'un dosage quantitatif on utilise des solutions standards (standards) de la troponine I cardiaque humaine purifiée [Larue C. et al, Clin. Chem. (1993), 39, p. 972-979].

L'invention concerne aussi l'utilisation du luminol et du lumigen PPD comme substrats chimiluminescents pour le dosage immunoenzymatique de type sandwich de la troponine I cardiaque mettant en oeuvre deux anticorps monoclonaux anti-troponine I cardiaque dont un couplé avec un marqueur enzymatique, en particulier la peroxydase, si le substrat est le luminol, et la phosphatase alcaline, si le substrat est le lumigen PPD, lesquels anticorps (soit naturellement, soit par coopérativité) ont pour la troponine I cardiaque une constante de dissociation à l'équilibre égale ou inférieure à $10^{-8}$ M.

L'invention a également pour objet une trousse de dosage de la troponine I cardiaque comprenant deux anticorps monoclonaux anti-troponine I cardiaque dont un est couplé avec un marqueur enzymatique lesquels anticorps, soit naturellement, soit par coopérativité ont pour la troponine I cardiaque une constante de dissociation à l'équilibre égale ou inférieure à $10^{-9}$ M, et un substrat chimiluminescent dérivé d'une diacylhydrazine, de préférence le luminol ou un dérivé de 1,2-dioxetane, de préférence le lumigen PPD.

Les exemples suivants, donnés à titre non limitatif, illustrent l'invention.

### EXEMPLE I

#### Dosage de la troponine I cardiaque avec un système automatisé

Le système automatisé utilisé pour le dosage immunoenzymatique est le système Access® immuoessai, système commercialisé par Sanofi Diagnostics Pasteur.

Le dosage est effectué de la manière suivante : Dans la cupule du dosage sont introduits 50 µl de l'échantillon à doser, 25 µl d'une solution d'immunoglobulines de souris à 4 mg/ml, 10 µl d'une solution d'acide succinique 0,1 M, 50 µl du conjugué anticorps monoclonal anti-troponine I cardiaque de souris 8E1-phosphatase alcaline (C = 5 µg/ml).

Après incubation pendant 5 minutes à 37°C sont introduits 50 µl de billes de latex ferriques (Rhône Poulenc réf. MI-070/60) sur lesquelles sont fixés par des liaisons covalentes des anticorps monoclonaux anti-troponine I cardiaque de souris 11E12.

Le mélange est incubé pendant 36 secondes à 37°C, puis les billes de latex ferriques sont séparées à l'aide d'un champ magnétique. On procède au lavage à l'aide d'une solution tampon Tris pH 8 on ajoute ensuite 200 µl de substrat Lumi-Phos™ 530.

La révélation est effectuée à 37°C et on mesure la luminescence générée par la réaction avec un luminomètre.

Le temps total d'analyse est de 15 minutes.

Pour la gamme d'étalonnage on utilise des solutions de la troponine I cardiaque humaine purifiée.

On effectue une gamme d'étalonnage correspondant à des concentrations comprises entre 0 et 50 µg/l.

La sensibilité du procédé, évaluée par la courbe d'étalonnage, est de 3,5 ng/l.

EP 0 804 736 B1

## EXEMPLE II

### Dosage de la troponine I cardiaque avec un kit immunoenzymatique

Dans des tubes en polystyrène revêtus d'anticorps monoclonaux anti-troponine cardiaque de la souris 8E1, on introduit 150 µl d'une solution tampon succinate contenant du Tween® 20 à 0,2 %, des immunoglobulines de souris non spécifiques et 0,1 % de Kathon, 50 µl du conjugué anticorps monoclonal anti-troponine I cardiaque de souris 11E1 2-peroxydase, et 200 µl d'échantillon à doser ou d'une solution étalon utilisée pour la gamme d'étalonnage.

Pour la gamme étalonnage on utilise du sérum humain contenant de 0 à 2 µg/l de troponine I cardiaque humaine purifiée.

On incube 15 minutes précisément sous agitation horizontale à température ambiante, puis on procède au lavage des tubes de la façon suivante :

- on renverse le contenu des tubes dans un récipient et on éponge les tubes sur un papier absorbant,
- on procède au lavage en ajoutant 1 ml de tampon phosphate 0,1 M, pH 6,8 contenant du Tween® 20 à 0,1 % et 0,3 % de Kathon, en maintenant la température au plus à 8°C. On répète cette étape 3 fois.

Après avoir procédé au lavage et éliminé toute trace de la soluticn utilisée pour ce dernier, la révélation enzymatique est effectuée en utilisant 300 µl d'un mélange constitué de 100 parties de luminol (BM chimiluminescent ELISA reagent Boehringer Mannheim) et 1 partie d'eau oxygénée.

On laisse à température ambiante et on mesure la luminescence avec un luminomètre après 3 minutes. Chaque tube est lu pendant exactement 30 secondes ou exactement 10 secondes.

La sensibilité de ce dosage est de 3 ng/l.

Les valeurs des rapports signal net/bruit de la mesure (S-B)/B obtenues selon le procédé décrit ci-dessus et selon le procédé décrit par Larue C. et al [Mol. Immunol. (1992), 29(2), p. 271-278 ; Clin Chem. (1993), 39/6 p. 972-979] sont données dans le Tableau II ci-après.

TABLEAU II

| Concentration en Troponine I cardiaque | Procédé l'exemple | Procédé décrit par Larue et al |
|---|---|---|
| 100 ng/l | $\frac{S-B}{B} = \frac{1200 - 95{:}95}{95} = 11,6$ | $\frac{S-B}{B} = \frac{150 - 50}{50} = 2,0$ |
| 10 ng/l | $\frac{S-B}{B} = \frac{250 - 95}{95} = 1,6$ | $\frac{S-B}{B} = 0 \qquad (S = B)$ |

Les valeurs des rapports signal/bruit de la mesure obtenue en appliquant le procédé de l'invention prouvent sa grande sensibilité et sa spécificité.

## Revendications

1. Procédé de dosage immunoenzymatique de type sandwich de la troponine I cardiaque caractérisé en ce que l'on met en oeuvre deux anticorps monoclonaux anti-troponine I cardiaque dont un est couplé avec un marqueur enzymatique lesquels anticorps, soit naturellement, soit par coopérativité ont pour la troponine I cardiaque une constante de dissociation à l'équilibre égale ou inférieure à $10^{-8}$ M, de préférence égale ou inférieure à $10^{-9}$ M et que l'on utilise pour la révélation enzymatique un substrat chimiluminescent dérivé d'une diacylhydrazine ou du 1,2-dioxetane.

2. Procédé selon la revendication 1, comprenant les étapes suivantes :

   a- les échantillons sont mis en contact avec un anticorps monoclonal anti-troponine I cardiaque couplé à un marqueur enzymatique et avec un deuxième anticorps monoclonal anti-troponine I cardiaque,
   b- la révélation enzymatique est effectuée par addition d'un substrat chimiluminescent dérivé d'une diacylhydrazine ou du 1,2-dioxetane,
   c- la quantité de la troponine I cardiaque est évaluée par la lecture directe du signal lumineux obtenu.

3. Procédé selon les revendications 1 ou 2 comprenant les étapes suivantes :

   a- les échantillons à doser ou les standards sont mis en contact simultanément avec un anticorps monoclonal anti-troponine I cardiaque couplé à un marqueur enzymatique et avec un deuxième anticorps monoclonal anti-troponine I cardiaque fixé éventuellement à un support solide,

b- le mélange réactionnel est incubé,

c- puis lavé,

d- la révélation enzymatique est effectuée par addition d'un substrat chimiluminescent, dérivé d'une diacylhydrazine ou dérivé du 1,2-dioxetane,

e- la quantité de la troponine I cardiaque présente dans des échantillons à doser ou les standards, est évaluée par la lecture directe du signal lumineux obtenu.

4.  Procédé selon les revendications 1 ou 2 comprenant les étapes suivantes :

    a- les échantillons à doser ou les standards sont mis en contact,

    -   soit, d'abord avec un anticorps monoclonal anti-troponine I cardiaque couplé à un marqueur enzymatique, puis après incubation, avec un deuxième anticorps monoclonal anti-troponine I cardiaque éventuellement fixé sur un support solide, et incubés de nouveau,
    -   soit, d'abord avec un anticorps monoclonal anti-troponine I cardiaque éventuellement fixé sur un support solide puis après incubation et lavage éventuel, avec un deuxième anticorps monoclonal anti-troponine I cardiaque couplé à un marqueur enzymatique et incubés à nouveau,

    b- le mélange réactionnel est ensuite éventuellement lavé,

    c- la révélation enzymatique est effectuée par addition d'un substrat chimiluminescent dérivé d'une diacylhydrazine ou dérivé de 1,2-dioxetane,

    d- la quantité de la troponine I cardiaque présente dans les échantillons à doser ou dans les standards est évaluée par la lecture directe du signal lumineux obtenu.

5.  Procédé selon l'une quelconque des revendications 1 à 4, caractérisé en ce que le substrat chimiluminescent est le luminol.

6.  Procédé selon l'une quelconque des revendications 1 à 4, caractérisé en ce que le substrat chimiluminescent est le lumigen PPD.

7.  Procédé selon l'une quelconque des revendications 1 à 4, caractérisé en ce que les anticorps monoclonaux anti-troponine I, sont des anticorps monoclonaux ayant les caractéristiques des anticorps monoclonaux de souris 11E12 et 8E1.

8.  Procédé selon l'une quelconque des revendications 1 à 4, caractérisé en ce que le marqueur enzymatique couplé à l'anticorps monoclonal anti-troponine I, est la phosphatase alcaline ou la peroxydase.

9.  Procédé selon l'une quelconque des revendications 1 à 3 caractérisé en ce que l'anticorps monoclonal anti-troponine I cardiaque couplé à un marqueur enzymatique est un conjugué anticorps monoclonal anti-troponine I cardiaque-peroxydase, ledit anticorps ayant les caractéristiques de l'anticorps monoclonal de souris 11E12 et le substrat chimiluminescent est un dérivé d'une diacylhydrazine.

10. Procédé selon l'une quelconque des revendications 1, 2 ou 4 caractérisé en ce que l'anticorps monoclonal anti-troponine I cardiaque couplé à un marqueur enzymatique est un conjugué anticorps monoclonal anti-troponine I cardiaque-phosphatase alcaline, ledit anticorps ayant les caractéristiques de l'anticorps monoclonal de souris 8E1, et le substrat chimiluminescent est un dérivé du 1,2-dioxetane.

11. Procédé selon l'une quelconque des revendications 1 à 5 caractérisé en ce que le substrat chimiluminescent est un mélange de luminol et de 4-iodophénol.

12. Procédé selon l'une quelconque des revendications 1 à 4 caractérisé en ce que la mise en contact des échantillons à doser ou des standards, avec un anticorps monoclonal anti-troponine I cardiaque couplé à un marqueur enzymatique et éventuellement à un deuxième anticorps anti-troponine I, est effectuée à un pH légèrement acide compris entre 5 et 6, de préférence entre 5,4 et 5,7.

13. Procédé selon l'une quelconque des revendications 1 à 4 caractérisé en ce que les solutions de lavage utilisées avant révélation ont un pH égal à 6,8-8 et une température de 2° à 37°C, de préférence à une température inférieure à 10°C.

14. Utilisation du luminol comme substrat chimiluminescent pour le dosage immunoenzymatique de type sandwich de la troponine I cardiaque mettant en oeuvre deux anticorps monoclonaux anti-troponine I cardiaque dont un couplé avec la peroxydase, lesquels anticorps soit naturellement soit par coopérativité, ont pour la troponine I cardiaque une constante de dissociation à l'équilibre égale ou inférieure à $10^{-8}$ M.

15. Utilisation du lumigen PPD comme substrat chimiluminescent pour le dosage immunoenzymatique de type sandwich de la troponine I cardiaque mettant en oeuvre deux anticorps monoclonaux anti-troponine I cardiaque dont un couplé avec la phosphatase alcaline, lesquels anticorps soit naturellement soit par coopérativité, ont pour la troponine I cardiaque une constante de dissociation à l'équilibre égale ou inférieure à $10^{-8}$ M.

16. Trousse de dosage de la troponine I cardiaque comprenant deux anticorps monoclonaux anti-troponine I cardiaque dont un est couplé avec un marqueur enzymatique lesquels anticorps, soit naturellement, soit par coopérativité ont pour la troponine I cardiaque une constante de dissociation à l'équilibre égale ou inférieure à $10^{-9}$ M, et un substrat chimiluminescent dérivé d'une diacylhydrazine ou du 1,2-dioxetane

**Patentansprüche**

1. Verfahren vom Typ des Sandwich-Tests zur immunoenzymatischen Bestimmung von Herz-Troponin I, dadurch gekennzeichnet, daß man zwei monoklonale Anti-Herz-Troponin I-Antikörper einsetzt, von denen einer mit einem enzymatischen Marker gekuppelt ist, welche Antikörper entweder von Natur aus oder durch Unterstützung eine Gleichgewichts-Dissoziationskonstante für Herz-Troponin I von gleich oder kleiner als $10^{-8}$ M, vorzugsweise gleich oder kleiner als $10^{-9}$ M aufweisen, und man zur enzymatischen Entwicklung ein von einem Diacylhydrazin oder von 1,2-Dioxetan abgeleitetes chemilumineszierendes Substrat verwendet.

2. Verfahren nach Anspruch 1 mil den folgenden Stufen:

   a - die Proben werden mit einem monoklonalen Anti-Herz-Troponin I-Antikörper, der mit einem enzymatischen Marker gekuppelt ist, und mit einem zweiten monoklonalen Anti-Herz-Troponin I-Antikörper in Kontakt gebracht,
   b - die enzymatische Entwicklung erfolgt durch zugabe eines chemilumineszierenden Substrats, das von einem Diacylhydrazin oder von einem 1,2-Dioxetan abgeleitet ist,
   c - die Menge des Herz-Troponins I wird durch direktes Ablesen des erhaltenen Lichtsignals gemessen.

3. Verfahren nach den Ansprüchen 1 oder 2 mit den folgenden Stufen:

   a - die zu bestimmenden Proben oder die Standards werden gleichzeitig mit einem monoklonalen Anti-Herz-Troponin I-Antikörper, der mit einem enzymatischen Marker gekuppelt ist, und mit einem zweiten monoklonalen Anti-Herz-Troponin I-Antikörper, der gegebenenfalls an einem festen Träger fixiert ist, in Kontakt gebracht,
   b - die Reaktionsmischung wird inkubiert,
   c - dann gewaschen,
   d - die enzymatische Entwicklung erfolgt durch Zugabe eines chemilumineszierenden Substrats, das von einem Diacylhydrazin abgeleitet ist oder von 1,2-Dioxetan abgeleitet ist,
   e - die Menge des in den zu bestimmenden Proben unter den Standards vorhandenen Herz-Troponins I wird durch direktes Ablesen des erhaltenen Lichtsignals gemessen.

4. Verfahren nach den Ansprüchen 1 oder 2 mit den folgenden Stufen:

   a - die zu bestimmenden Proben oder die Standards werden

   - entweder zunächst mit einem monoklonalen Anti-Herz-Troponin I-Antikörper, der mit einem enzymatischen Marker gekuppelt ist und dann nach der Inkubation mit einem zweiten monoklonalen Anti-Herz-Troponin I-Antikörper, der gegebenenfalls an einem festen Träger fixiert ist, in Kontakt gebracht und erneut inkubiert.
   - oder zunächst mit einem monoklonalen Anti-Herz-Troponin I-Antikörper, der gegebenenfalls an einem festen Träger fixiert ist und dann nach der Inkubation und dem eventuellen Waschen mit einem zweiten monoklonalen Anti-Herz-Troponin I-Antikörper, der mit einem enzymatischen Marker gekuppelt ist, in Kontakt gebracht und erneut inkubiert,

b - die Reaktionsmischung wird anschließend gegebenenfalls gewaschen,
c - die enzymatische Entwicklung erfolgt durch Zugabe eines chemilumineszierenden Substrats, welches von einem Diacylhydrazin abgeleitet ist oder welches von 1,2-Dioxetan abgeleitet ist.
d - die Menge des in den zu bestimmenden Proben oder in den Standards vorhandenen Herz-Troponins I wird durch direktes Ablesen des erhaltenen Lichtsignals gemessen.

5. Verfahren nach irgendeinem der Ansprüche 1 bis 4, dadurch gekennzeichnet, daß das chemilumineszierende Substrat Luminol ist.

6. Verfahren nach irgendeinem der Ansprüche 1 bis 4, dadurch gekennzeichnet, daß das chemilumineszierende Substrat Lumigen PPD ist.

7. Verfahren nach irgendeinem derAnspruche 1 bis 4, dadurch gekennzeichnet, daß die monoklonalen Anti-Troponin I-AntikOrper monoklonale Antikörper sind, welche die Eigenschaften der monoklonalen Mäuse-Antikörper 11E12 und 8E1 besitzen.

8. Verfahren nach irgendeinem der Ansprüche 1 bis 4, dadurch gekennzeichnet, daß der mit dem monoklonalen Anti-Troponin I-Antikörper gekuppelte enzymatische Marker alkalische Phosphatase oder Peroxidase ist.

9. Verfahren nach irgendeinem der Ansprüche 1 bis 3, dadurch gekennzeichnet, daß der an einen enzymatischen Marker gekuppelte monoklonale Anti-Herz-Troponin I-Antikörper ein Konjugat aus dem monoklonalen Anti-Herz-Troponin I-Antikörper und Peroxidase ist, wobei die Antikörper die Eigenschaften des monoklonalen Mäuse-Antikörpers 11E12 besitzen, und das chemilumineszierende Substrat ein Derivat eines DIacylhydrazins ist.

10. Verfahren nach irgendeinem der Ansprüche 1 , 2 oder 4, dadurch gekennzeichnet, daß der monoklonale Anti-Herz-Troponin 1-Antikörper, der mit einem enzymatischen Marker gekuppelt ist, ein Konjugat aus dem monoklonalen Anti-Herz-Troponin I-Antikörper und alkalischer Phosphatase ist, welche Antikörper die Eigenschaften des monoklonalen Mäuse-Antikörpers 8E1 besitzen. und das chemilumineszierende Substrat ein Derivat von 1,2-Dioxetan ist.

11. Verfahren nach irgendeinem der Ansprüche 1 bis 5, dadurch gekennzeichnet, daß das chemilumineszierende Substrat eine Mischung aus Luminol und 4-Iodphenol ist.

12. Verfahren nach irgendeinem der Ansprüche 1 bis 4, dadurch gekennzeichnet, daß das Inkontaktbringen der zu bestimmenden Proben oder der Standards mit einem monoklonalen Anti-Herz-Troponin I-Antikörper, der mit einem enzymatischen Marker gekuppelt ist, und gegebenenfalls einem zweiten Anti-Tropanin I-Antikörper bei einem schwach sauren pH-Wert zwischen 5 und 6, vorzugsweise zwischen 5,4 und 5,7, erfolgt.

13. Verfahren nach irgendeinem der Ansprüche 1 bis 4, dadurch gekennzeichnet. daß die vor der Entwicklung verwendeten Waschlösungen einen pH-Wert von 6.8 bis 8 und eine Temperatur von 2° bis 37°C, vorzugsweise eine Temperatur von unterhalb 10°C aufweisen.

14. Verwendung von Luminol als chemilumineszierendes Substrat für die immunoenzymatische Bestimmung von Herz-Troponin I nach einer Methode vom Typ des Sandwich-Tests unter Verwendung von zwei monoklonalen Anti-Herz-Troponin I-Antikörpern, von denen einer mit Peroxidase gekuppelt ist, welche Antikörper entweder von Natur aus oder durch Unterstützung eine Gleichgewichts-Dissoziationskonstante für Herz-Troponin I von gleich oder kleiner $10^{-8}$ M besitzen.

15. Verwendung von Lumigen PPD als chemilumineszierendes Substrat für die immunoenzymatische Bestimmung von Herz-Troponln I nach einer Methode vom Typ des Sandwich-Tests unter Verwendung von zwei monoklonalen Anti-Herz-Troponin I-Antikörpern, von denen einer mit Peroxidase gekuppelt ist, welche Antikörper entweder natürlich oder durch Unterstützung eine Gleichgewichts-Dissoziationskonstante für Herz-Troponin I von gleich oder kleiner $10^{-8}$ M besitzen.

**16.** Kit zur Bestimmung von Herz-Troponin I umfassend zwei monoklonale Anti-Herz-Troponin I-Antikörper, von denen einer mit einem enzymatischen Marker gekuppelt ist, welche Antikörper entweder von Natur aus oder durch Unterstützung eine Gleichgewichts-Dissoziationskonstünte für Herz-Troponin I von gleich oder kleiner $10^{-9}$ M aufweisen, und ein chemilumineszierendes Substrat, welches von einem Diacylhydraxin oder von 1,2-Dioxetan abgeleitet ist.

## Claims

**1.** Process for sandwich-type immunoenzymatic titration of cardiac troponin I, characterised in that two monoclonal anti-cardiac-troponin I antibodies are used, one of which is coupled to an enzymatic marker, said antibodies having an equilibrium dissociation constant for cardiac troponin I, either naturally or by cooperativity, less than or equal to $10^{-8}$ M, preferably less than or equal to $10^{-9}$ M, and that a chemiluminescent substrate derived from a diacylhydrazine or from 1,2-dioxetane is used for the enzymatic development.

**2.** Process according to claim 1, comprising the following steps:

a - the samples are brought into contact with a monoclonal anti-cardiac troponin I antibody coupled to an enzymatic marker and with a second monoclonal anti-cardiac troponin I antibody,
b - the enzymatic development is carried out by the addition of a chemiluminescent substrate derived from a diacylhydrazine or from 1,2-dioxetane,
c - the quantity of cardiac troponin I is evaluated by directly reading off the light signal obtained.

**3.** Process according to claim 1 or 2, comprising the following steps:

a - the samples to be titrated or the standards are brought into contact simultaneously with a monoclonal anti-cardiac troponin I antibody coupled to an enzymatic marker and with a second monoclonal anti-cardiac troponin I antibody optionally fixed to a solid support,
b - the reaction mixture is incubated,
c - then washed,
d - the enzymatic development is carried out by the addition of a chemiluminescent substrate derived from a diacylhydrazine or from 1,2-dioxetane,
e - the quantity of cardiac troponin I present in the samples to be titrated or in the standards is evaluated by directly reading off the light signal obtained.

**4.** Process according to claim 1 or 2 comprising the following steps:

a - the samples to be titrated or the standards are brought into contact

- either first of all with a monoclonal anti-cardiac troponin I antibody coupled to an enzymatic marker then, after incubation, with a second monoclonal anti-cardiac troponin I antibody optionally fixed to a solid support, and incubated again,
- or first of all with a monoclonal anti-cardiac troponin I antibody optionally fixed to a solid support then, after incubation and optional washing, with a second monoclonal anti-cardiac troponin I antibody coupled to an enzymatic marker and incubated again,

b - the reaction mixture is then optionally washed,
c - the enzymatic development is carried out by the addition of a chemiluminescent substrate derived from a diacylhydrazine or from 1,2-dioxetane,
d - the quantity of cardiac troponin I present in the samples to be titrated or in the standards is evaluated by directly reading off the light signal obtained.

**5.** Process according to any one of claims 1 to 4, characterised in that the chemiluminescent substrate is luminol.

**6.** Process according to any one of claims 1 to 4, characterised in that the chemiluminescent substrate is the lumigen PPD.

**7.** Process according to any one of claims 1 to 4, characterised in that the monoclonal anti-troponin I antibodies are monoclonal antibodies having the characteristics of the monoclonal murine antibodies 11E12 and 8E1.

8. Process according to any one of claims 1 to 4, characterised in that the enzymatic marker coupled to the monoclonal anti-troponin I antibody is alkaline phosphatase or peroxidase.

9. Process according to any one of claims 1 to 3, characterised in that the monoclonal anti-cardiac troponin I antibody coupled to an enzymatic marker is a monoclonal anti-cardiac troponin I antibody/peroxidase conjugate, said antibody having the characteristics of the monoclonal murine antibody 11E12, and the chemiluminescent substrate is a derivative of a diacylhydrazine.

10. Process according to any one of claims 1, 2 or 4, characterised in that the monoclonal anti-cardiac troponin I antibody coupled to an enzymatic marker is a monoclonal anti-cardiac troponin I antibody/alkaline phosphatase conjugate, said antibody having the characteristics of the monoclonal murine antibody 8E1, and the chemiluminescent substrate is a derivative of 1,2-dioxetane.

11. Process according to any one of claims 1 to 5, characterised in that the chemiluminescent substrate is a mixture of luminol and 4-iodophenol.

12. Process according to any one of claims 1 to 4, characterised in that the contacting of the samples to be titrated or the standards with a monoclonal anti-cardiac troponin I antibody coupled to an enzymatic marker and optionally with a second anti-troponin I antibody is carried out at a slightly acid pH between 5 and 6, preferably between 5.4 and 5.7.

13. Process according to any one of claims 1 to 4, characterised in that the washing solutions used before developing have a pH equal to 6.8 - 8 and are at a temperature of from 2° to 37°C, preferably at a temperature below 10°C.

14. Use of luminol as the chemiluminescent substrate for the sandwich-type immunoenzymatic titration of cardiac troponin I, using two monoclonal anti-cardiac-troponin I antibodies, one of which is coupled to peroxidase, said antibodies having an equilibrium dissociation constant for cardiac troponin I, either naturally or by co-operativity, less than or equal to $10^{-8}$ M.

15. Use of lumigen PPD as the chemiluminescent substrate for the sandwich-type immunoenzymatic titration of cardiac troponin I, using two monoclonal anti-cardiac-troponin I antibodies, one of which is coupled to alkaline phosphatase, said antibodies having an equilibrium dissociation constant for cardiac troponin I, either naturally or by co-operativity, less than or equal to $10^{-8}$ M.

16. Kit for titration of cardiac troponin I comprising two monoclonal anti-cardiac-troponin I antibodies, one of which is coupled to an enzymatic marker, said antibodies having an equilibrium dissociation constant for cardiac troponin I, either naturally or by co-operativity, less than or equal to $10^{-9}$ M, and a chemiluminescent substrate derived from a diacylhydrazine or from 1,2-dioxetane.